# EUROPEAN PATENT APPLICATION

(11) **EP 3 693 045 A1**
(43) Date of publication of application: **12.08.2020**
(21) Application number: 20167755.6
(22) Date of filing: 21.03.2017
(51) Int. Cl.: A61M 15/06, A24F 47/00, A61M 11/04, A61M 15/00

(54) **ELECTRONIC VAPOUR PROVISION SYSTEM**

(30) Priority: 24.03.2016 GB 201605106
(62) Divisional of application: 17714514.1
(71) Applicant: Nicoventures Holdings Limited, London WC2R 3LA (GB)
(72) Inventor: NETTENSTROM, Matthew Joe, Streamwood, IL 60181 (US); MCKEON, Thomas Michael, Streamwood, IL 60181 (US); SCHENNUM, Steven Michael, Streamwood, IL 60181 (US); PEART, Justin Banker, Streamwood, IL 60181 (US)
(74) Representative: D Young & Co LLP

(57) **Abstract**

A vapour provision device comprising a vaporiser for generating a vapour from a vapour precursor material for inhalation by a user; wherein the device has a length L along a length direction, a thickness T along a thickness direction which is orthogonal to the length direction, and a width W along a width direction which is perpendicular to both the length direction and the thickness direction, wherein the width W and length L are both at least twice the thickness T, and wherein a minimum radius of curvature for a peripheral edge of the device in a plane perpendicular to the thickness direction is at least 0.1 W.

## Description

### Field

The present disclosure relates to electronic vapour provision systems such as nicotine delivery systems (e.g. electronic cigarettes and the like), and in particular to shapes for such systems.

### Background

Electronic vapour provision systems such as electronic cigarettes (e-cigarettes) generally contain a vapour precursor material, such as a reservoir of a source liquid containing a formulation, typically including nicotine, or a solid material such a tobacco-based product, from which a vapour is generated for inhalation by a user, for example through heat vaporisation. Thus, a vapour provision system will typically comprise a vapour generation chamber containing a vaporiser, e.g. a heating element, arranged to vaporise a portion of precursor material to generate a vapour in the vapour generation chamber. As a user inhales on the device and electrical power is supplied to the vaporiser, air is drawn into the device through inlet holes and into the vapour generation chamber where the air mixes with the vaporised precursor material. There is a flow path connecting between the vapour generation chamber and an opening in the mouthpiece so the incoming air drawn through the vapour generation chamber continues along the flow path to the mouthpiece opening, carrying some of the vapour with it, and out through the mouthpiece opening for inhalation by the user.

It is common for vapour provision systems to comprise two main functional parts, namely a reusable part and disposable / replaceable cartridge part. Typically the cartridge part will comprise the consumable vapour precursor material and the vaporiser, while the reusable device part will comprise longer-life items, such as a rechargeable battery, device control circuitry, activation sensors and user interface features. The reusable part may also be referred to as a control unit or battery section and the replaceable cartridge part may also be referred to as a cartomiser.

The control unit and cartomiser are mechanically coupled together at an interface for use, for example using a screw thread or bayonet fixing. When the vapour precursor material in a cartomiser is exhausted, or the user wishes to switch to a different cartomiser having a different vapour precursor material, the cartomiser may be removed from the control unit and a replacement cartomiser may be attached to the device in its place.

Electronic cigarettes typically comprise a generally cylindrical configuration having a degree of circular symmetry about a longitudinal axis. However, other configurations are known, for example shapes comprising a box-like reusable part with a cylindrical cartomiser attached.

The inventors have recognised certain drawbacks with existing configurations for electronic cigarettes, for example in terms of ease and comfort of handling and restrictions on available space for internal components, such as a battery. Alternative configurations for vapour provision systems, such as electronic cigarettes, are therefore of interest.

### Summary

According to a first aspect of certain embodiments there is provided a vapour provision device comprising a vaporiser for generating a vapour from a vapour precursor material for inhalation by a user; wherein the device has a length L along a length direction, a thickness T along a thickness direction which is orthogonal to the length direction, and a width W along a width direction which is perpendicular to both the length direction and the thickness direction, wherein the width W and length L are both at least twice the thickness T, and wherein a minimum radius of curvature R for a peripheral edge of the device in a plane perpendicular to the thickness direction is at least 0.1 times the width W.

In accordance with some embodiments, the length L is greater than the thickness T by a factor of at least 2, at least 2.5, at least 3, at least 3.5, at least 4, at least 4.5, or at least 5.

In accordance with some embodiments, the width W is greater than the thickness T by a factor of at least 2, at least 2.5, at least 3, at least 3.5, at least 4, at least 4.5, or at least 5.

In accordance with some embodiments, the length L is greater than the width by a factor of at least 1.25, at least 1.3, at least 1.5, at least 2, at least 2.5, or at least 3.

In accordance with some embodiments, the thickness T is less than 25 mm, less than 22mm, less than 20 mm, less than 18 mm, less than 16 mm, less than 14 mm, less than 12 mm, or less than 10 mm.

In accordance with some embodiments, the width is greater than 20 mm, greater than 25 mm, greater than 30 mm, greater than 35 mm, greater than 40 mm, greater than 45 mm, or greater than 50 mm.

In accordance with some embodiments, the length is less than 120 mm, less than 110 mm, less than 100 mm, less than 90 mm, or less than 80 mm.

In accordance with some embodiments, L is between 60 mm and 100 mm, or more preferably L is between 70 mm and 90 mm; and / or W is between 30 mm and 45 mm, or more preferably between 35 mm and 40 mm; and / or T is between 12 mm and 20 mm, or more preferably between 15 mm and 17 mm.

In accordance with some embodiments, the minimum radius of curvature R for a peripheral edge of the device in the plane perpendicular to the thickness direction is at least 0.2 times the width W, at least 0.3 times the width W, at least 0.4 times the width W, or at least 0.5 times the width W.

In accordance with some embodiments, the minimum radius of curvature R for a peripheral edge of the device in the plane perpendicular to the thickness direction is at least 3 mm, at least 4 mm, at least 5 mm, at least 6 mm, at least 7 mm, at least 8 mm, at least 9 mm or at least 10 mm.

In accordance with some embodiments, an areal extent of the device in the plane perpendicular to the thickness direction is less than the product of width and the length by a factor of less than 0.95, less than 0.9, less than 0.85, and less than 0.8.

In accordance with some embodiments, at least one of the surfaces of the device perpendicular to the thickness direction is curved in the width direction along a majority of the width of the device.

In accordance with some embodiments, at least one of the surfaces of the device perpendicular to the thickness direction is curved in the length direction along a majority of the length of the device.

In accordance with some embodiments, at least one of the sides of the device perpendicular to the width direction is curved in the length direction along a majority of the length of the device.

In accordance with some embodiments, at least one of the ends of the device perpendicular to the width direction is curved in the width direction along a majority of the width of the device.

In accordance with some embodiments, an outer surface of the device is provided with at least one depression having a depth at its deepest part of between 1 mm and 5 mm, or between 2 mm and 4 mm, and a width of between 0.2W and 0.8W, between 0.25W and 0.75W, between 0.3W and 0.7W, between 0.35W and 0.65W, between 0.4W and 0.6W, or between 0.45W and 0.65W.

In accordance with some embodiments, the device comprises a control unit and a detachable cartridge, wherein the cartridge comprises the vapour precursor material and the control unit comprises a power supply for supplying power to the vaporiser to selectively generate the vapour from vapour precursor material.

In accordance with some embodiments, the detachable cartridge further comprises the vaporiser.

In accordance with some embodiments, the vapour precursor material comprises a liquid formulation.

According to another aspect of certain embodiments there is provided a vapour provision device comprising a vaporiser for generating a vapour from a vapour precursor material for inhalation by a user; wherein a majority of the outer surface of the device is curved.

According to another aspect of certain embodiments there is provided a vapour provision device comprising a vaporiser for generating a vapour from a vapour precursor material, wherein the device has a length L along a length direction, a thickness T along a thickness direction which is orthogonal to the length direction, and a width W along a width direction which is perpendicular to both the length direction and the thickness direction, wherein the width W and length L are both at least twice the thickness T, and wherein a majority of the a peripheral edge of the device in a plane perpendicular to the thickness direction is curved.

These and further aspects of certain embodiments are set out in the appended independent and dependent claims. It will be appreciated that features of the dependent claims may be combined with each other and features of the independent claims in combinations other than those explicitly set out in the claims. Furthermore, the approaches described herein are not restricted to specific embodiments such as the examples set out below, but include and contemplate any appropriate combinations of features presented herein. For example, a vapour provision system may be provided in accordance with approaches described herein which includes any one or more of the various features described below as appropriate.

### Brief Description of the Drawings

Embodiments of the invention will now be described, by way of example only, with reference to the accompanying drawings, in which:
Figure 1 schematically represents a cross-section view of a vapour provision system in accordance with certain embodiments of the disclosure;
Figure 2 schematically represents in perspective view the outer form of the vapour provision system represented in Figure 1;
Figures 3A and 3B schematically represent respective top and bottom views of the vapour provision system of Figure 2;
Figures 4A and 4B schematically represent respective side views of the vapour provision system of Figure 2;
Figures 5A and 5B schematically represent respective end views of the vapour provision system of Figure 2;
Figures 6 to 12 schematically represent views of generally flat and rounded vapour provision systems in accordance with some other example embodiments of the present disclosure.

### Detailed Description

Aspects and features of certain examples and embodiments are discussed / described herein. Some aspects and features of certain examples and embodiments may be implemented conventionally and these are not discussed / described in detail in the interests of brevity. It will thus be appreciated that aspects and features of apparatus and methods discussed herein which are not described in detail may be implemented in accordance with any conventional techniques for implementing such aspects and features.

The present disclosure relates to aerosol provision systems, also referred to as vapour provision systems, such as e-cigarettes. Throughout the following description the term "e-cigarette" or "electronic cigarette" may sometimes be used; however, it will be appreciated this term may be used interchangeably with vapour provision system and electronic vapour provision system. Furthermore, and as is common in the technical field, the terms "vapour" and "aerosol", and related terms such as "vaporise" and "aerosolise", may also be used interchangeably.

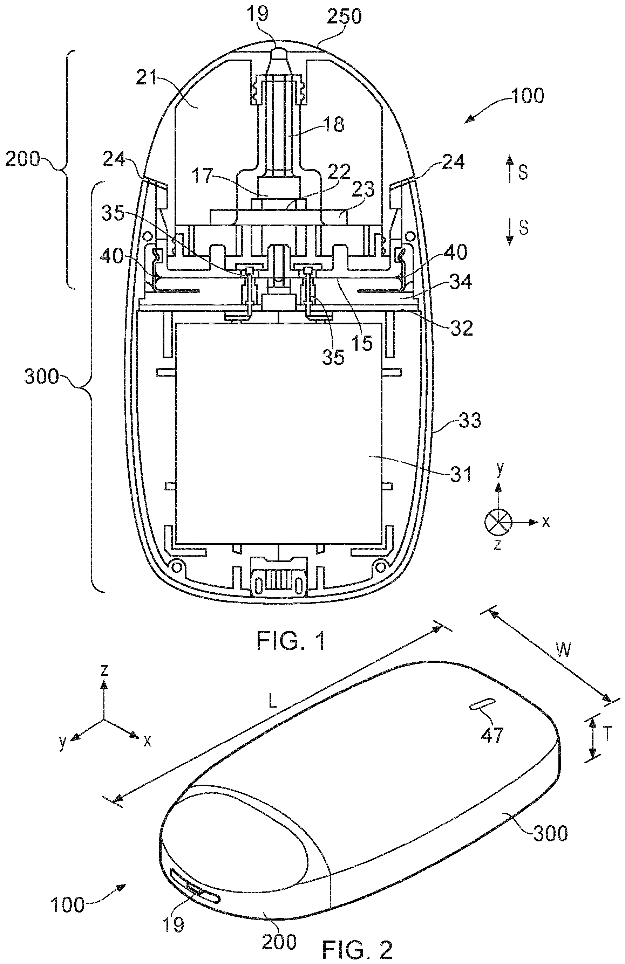

Figure 1 is a cross-sectional view through an example e-cigarette 100 in accordance with some embodiments of the disclosure. The e-cigarette 100 comprises two main components, namely a cartomiser 200 and a control unit 300.

The cartomiser 200 includes a reservoir 21 containing a supply of liquid, a heater 22 to act as an atomiser or vaporiser, and a mouthpiece 250. In this example the heater 22 comprises a nickel chrome alloy (Cr20Ni80) wire. The liquid in the reservoir 21 (sometimes referred to as the e-liquid or source liquid) typically includes nicotine in an appropriate solvent, and may include further constituents, for example, to aid aerosol formation, and / or for additional flavouring. The cartomiser 200 further includes a wick 23, which in this example comprises a glass fibre bundle, or a similar facility to transport an amount of liquid from the reservoir 21 to a heating location on or adjacent the heater 22. The vaporiser (heater) 22 is located in a vapour generation chamber 17. The vapour generation chamber 17 is arranged in an air flow path that extends from air inlets / ventilation slots 24 provided at the joint between the cartomiser 200 and control unit 300, into the cartomiser 200 and through the vapour generation chamber 17 past the heater (vaporiser) 22, and along an air channel 18 providing fluid communication between the vapour generation chamber 17 and a vapour outlet 19 provided in the mouthpiece 250.

The control unit 300 includes within a housing 33 a re-chargeable cell or battery 31 to provide power to the e-cigarette 100 and a control printed circuit board 32 (PCB) comprising circuitry for generally controlling the operation of the e-cigarette, which may be undertaken in accordance with generally conventional techniques. The rechargeable battery 31 may be charged through a charging port 37, e.g. a USB-based charging port, in accordance with conventional techniques.

Although not apparent in Figure 1, the control unit may comprise further circuit boards for providing functionality associated with the operation of the aerosol provision system. When the heater 22 receives power from the battery 31, e.g. as controlled by the control PCB 32, the heater 22 vaporises a portion of liquid from the wick 23 to create a vapour in the vapour generation chamber 17, which is mixed with incoming air from the ventilation slots 24 and drawn along the air channel 18 and out through the vapour outlet 19 into the mouth of a user inhaling on the e-cigarette 100.

For ease of reference and further explanation, a Cartesian coordinate system defined by X-, Y- and Z- axes is included in Figure 1. This coordinate system is arranged so the X-axis corresponds to a width direction for of the e-cigarette (extending from left to right for the orientation in Figure 1), the Y-axis corresponds to a length direction for the e-cigarette (extending from bottom to top for the orientation shown in Figure 1), and the Z- axis corresponds to a thickness direction for of the e-cigarette (extending from front to back for the orientation in Figure 1).

The cartomiser 200 and the control unit 300 are detachable from one another by separation in a direction parallel to the Y-axis, indicated in Figure 1 by the arrows S, but are joined together (as in Figure 1) when the device 100 is in use so as to provide mechanical and electrical connectivity between the cartomiser 200 and the control unit 300. The mechanical connection is facilitated by latching elements 40. When the e-liquid in the cartomiser reservoir 21 has been depleted, or the user wishes to switch to a different cartomiser, for example containing a different flavour vapour precursor material, the cartomiser 200 is removed and a new cartomiser is attached to the control unit 300. Accordingly, the cartomiser 200 may sometimes be referred to as a disposable portion of the e-cigarette 100, while the control unit 300 represents a re-usable portion. Alternatively, the cartomiser may be configured to be refillable with e-liquid, and may require detachment from the control unit for access to a filling port.

The e-cigarette 100 includes a sealing member or seal 34 disposed at a generally planar physical interface 15 between the control unit 300 and the cartomiser 200 when the two components are connected together for use. In this example the seal 34 is disposed within the control unit 300, over the control PCB 32. The seal 34 is fabricated from a resilient compressible material such as silicone, rubber, sponge, cork or a flexible plastic, and sized (along the Y-axis) so as to undergo a degree of resilient compression when the cartomiser 200 and the control unit 300 are joined together and extends (along the X- and Z-axes) generally to the interior of the side walls of the control unit housing 33. The seal 34 thus helps to provides a secure and close fit between the control unit 300 and the cartomiser 200 while also applying a biasing force along the Y-axis (due to its resilient compression) at the mechanical interface between the cartomiser and the controller unit when they are connected together. An outer surface of the seal 34 (i.e. the surface facing the cartomiser) comprises channels forming part of the fluid communication path between the air inlet / ventilation slots 24 and vaporisation chamber / vapour generation chamber 17.

The seal 34 has through-apertures to receive conductive connectors in the form of the sprung pins 35 that provide electrical connection between the control unit and the cartomiser when coupled together as discussed further below. The sprung pins ("pogo pins") 35 are, in this example, mounted to the circuit board 32 and may be provided in accordance with conventional techniques for providing such connectors.

When a user inhales through the mouthpiece 250 the vapour generation function of the electronic cigarette is activated - i.e. electrical power is supplied to the vaporiser / heater 22. The activation of the vapour generation function may be based on conventional techniques, for example a user-activated button or an inhalation sensor, for example based around a pressure sensor / microphone arranged to detect a drop in pressure when a user inhales on the device, may be used. These, and other, conventional operating aspects of aerosol provision systems in accordance with the principles described herein may be provided in accordance with conventional techniques and are not described further.

As the user inhales on the mouthpiece 250, air flows into the cartomiser 200 through the air inlet hole 214 (via a pathway leading from ventilation slots 24 defined at the juncture between the outer edges of the control unit 300 and cartomiser / cartridge 200. This incoming air flows past the heater which receives electrical power from the battery in the control unit 300 so as to vaporise liquid from the reservoir 21 (and more especially from the wick 23). This vaporised liquid is then incorporated / entrained into the airflow through the cartomiser, and drawn out of the cartomiser 200 through mouthpiece 250 for inhalation by the user.

Figure 2 is an external perspective view of the e-cigarette 100 of Figure 1, in its assembled configuration with the cartomiser 200 coupled to the control unit 300 so that the e-cigarette is ready for use. Also apparent in Figure 2 is a combined button / indicator light 47, the button function allows for user input control, e.g. to activate the atomiser, and the indicator light function allows for status feedback for the user, e.g. to indicate when the device is, or is ready, for use.

The orientation represented in Figure 2 relative to the view of Figure 1 is apparent from the representation of the X-, Y- and Z-axes. As indicated in Figure 2, the Z-axis (thickness direction) is parallel to a direction along which the electronic cigarette has its minimum extent, the Y-axis is parallel to a direction along which the electronic cigarette has its maximum extent ad which is perpendicular to the thickness direction, and the X-axis is parallel to a direction along which the electronic cigarette has its maximum extent in a direction which is perpendicular to both the thickness direction and the length direction.

As schematically indicated in Figure 2, the electronic cigarette 100 has a maximum extent along the Z-axis (i.e. a thickness) of T, a maximum extent along the X-axis (i.e. a width) of W, and a maximum extent along the Y-axis (i.e. a length) of L. The coordinate system defined by the X-, Y-, and Z-axes in this example is such that the X-axis increases from left to right for the orientation shown in Figure 1, the Y-axis increases from bottom to top (i.e. from the charging port 37 end of the device to the mouthpiece / vapour exit end of the device) for the orientation shown in Figure 1, and the Z-axis increases from above the plane of Figure 1 to below the plane of Figure 1.

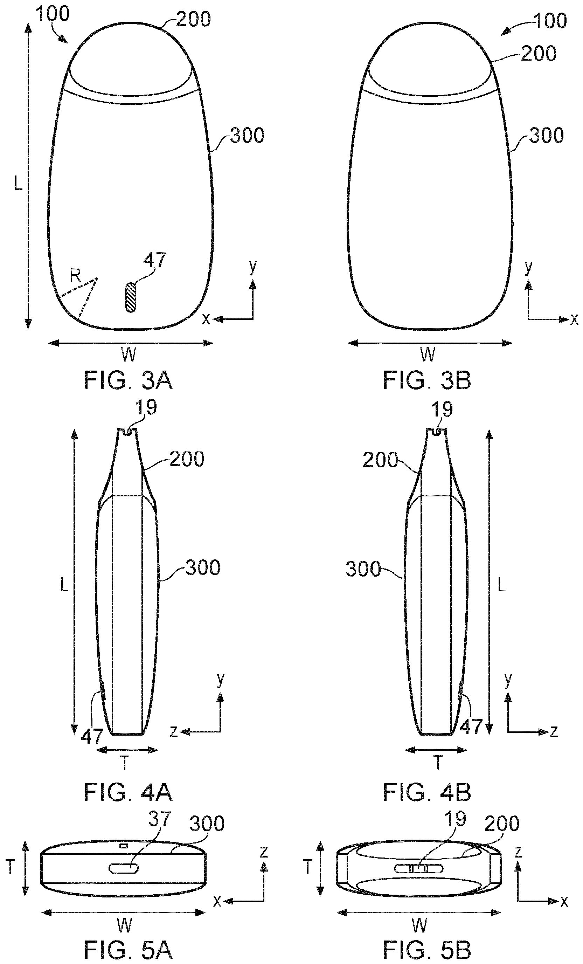

Figure 3A is a schematic view of the e-cigarette 100 in the XY plane viewed along the decreasing Z-direction, i.e. in what may be referred to here as a top-view (i.e. showing the combined light / button 47). The orientation of the X- and Y- axes is as shown in the figure.

Figure 3B is a schematic view of the e-cigarette in the XY plane viewed along the increasing Z-direction, i.e. in what may be referred to here as a bottom-view (i.e. not showing the combined light / button 47). The orientation of the X- and Y- axes is as shown in the figure.

Figure 4A is a schematic view of the e-cigarette in the YZ plane viewed along the increasing X-direction, i.e. in what may be referred to here as a left side-view. The orientation of the Y- and Z- axes is as shown in the figure.

Figure 4B is a schematic view of the e-cigarette in the YZ plane viewed along the decreasing X-direction, i.e. in what may be referred to here as a right side-view. The orientation of the Y- and Z- axes is as shown in the figure.

Figure 5A is a schematic view of the e-cigarette in the XZ plane viewed along the increasing Y-direction, i.e. in what may be referred to here as a charging port end view (i.e. showing the charging port 37). The orientation of the X- and Z- axes is as shown in the figure.

Figure 5B is a schematic view of the e-cigarette in the XZ plane viewed along the decreasing Z-direction, i.e. in what may be referred to here as a mouthpiece end view (i.e. showing the vapour outlet 19). The orientation of the X- and Z- axes is as shown in the figure.

As can be seen from Figures 2 to 5, the overall shape / outline / form of the electronic cigarette 100 differs significantly from known configurations. In particular, the thickness T is significantly less than both the length L and width W. Furthermore, the shape / outline / form of the electronic cigarette in a plane perpendicular to the thickness direction is generally rounded / smooth (i.e. does not have significant corners). That is to say, there is a minimum radius of curvature R for the outline of the electronic cigarette 100 comprising the assembled cartomiser 200 and control unit 300 in the plane perpendicular to the thickness direction (which for the specific device represented in Figure 3A is at the lower left and lower right corner) which is greater than a minimum threshold value which is relatively large compared to other characteristic dimensions of the electronic cigarette 100. For example, the minimum radius of curvature R for the outline of the electronic cigarette in the plane perpendicular to the thickness may be a significant fraction of (e.g. 0.5 or larger) than the thickness. In some examples the majority of the outline of the electronic cigarette in the plane perpendicular to the thickness may be non-flat / curved. In yet other examples, despite the device as whole having a generally flat configuration, the majority of its entire outer surface may be non-flat / curved.

Thus, and as is most apparent in Figures 4 and 5, the upper and lower faces of the electronic cigarette, i.e. the faces which are generally perpendicular to the thickness direction, are not flat in the plane perpendicular to the thickness direction, but curve both along the width direction (as seen in Figures 5A and 5B) and the length direction (as seen in Figure 4A and 4B) across a majority of the respective surfaces. Furthermore, the size of the electronic cigarette, i.e. the faces which are generally perpendicular to the width direction, are also not flat in the plane perpendicular to the width direction, but curve along the length direction along a majority of the length.

This configuration results in the e-cigarette 100 having a generally flat or planar configuration (with the two largest opposing surfaces extending generally parallel to the XY plane) and having a generally smooth / rounded overall shape. The inventors have recognised this generally flat and rounded shape is convenient and comfortable for users to hold, while still providing a relatively large volume for a given maximum extent (i.e. length), thereby allowing, for example, a correspondingly relatively large battery in an otherwise compact device. Furthermore, the generally flat / planar configuration can allow for a layer-like construction, for example with a control circuit board arranged adjacent (in the thickness direction) a generally flat battery, and this can in some respects simplify assembly, for example by reducing the requirement for axial and rotational alignment of the layered components.

By way of a specific example size, the electronic cigarette represented in Figures 1 to 5 may have a length L (along the Y-axis) of around 70 mm, a width W (along the X-axis) of around 35 mm and a thickness T (along the Z-axis) of around 14 mm. i.e. the width W in this example is around 2.5 times the thickness T, and the length L is around 5 times the thickness T. Furthermore, the minimum radius of curvature R for the outline of the device in a plane perpendicular to the thickness direction in this example is around 7 mm (i.e. around one tenth the length (i.e. 0.1 L); equivalent to around one fifth of the width (i.e. 0.2 W); equivalent to around half the thickness (i.e. 0.5 T). In addition, the curvature of the largest surfaces of the electronic cigarette are such that the thickness of the cigarette at its perimeter in the plane perpendicular to the thickness direction is around half the maximum thickness of the device (i.e. the thickness T around the perimeter of the device is around half the thickness T around the middle of the device in the XY plane).

However, it will of course be appreciated the principles described herein may be equally applied to electronic cigarettes having generally different sizes and shapes. In accordance with certain embodiments, what is significant is not the specific size and shape, but that the device has a thickness less than both its width and length, and its outline in a plane perpendicular to the thickness direction has a minimum radius of curvature as discussed above, i.e. so that the device is characterised by a generally curved / smooth shape in this plane.

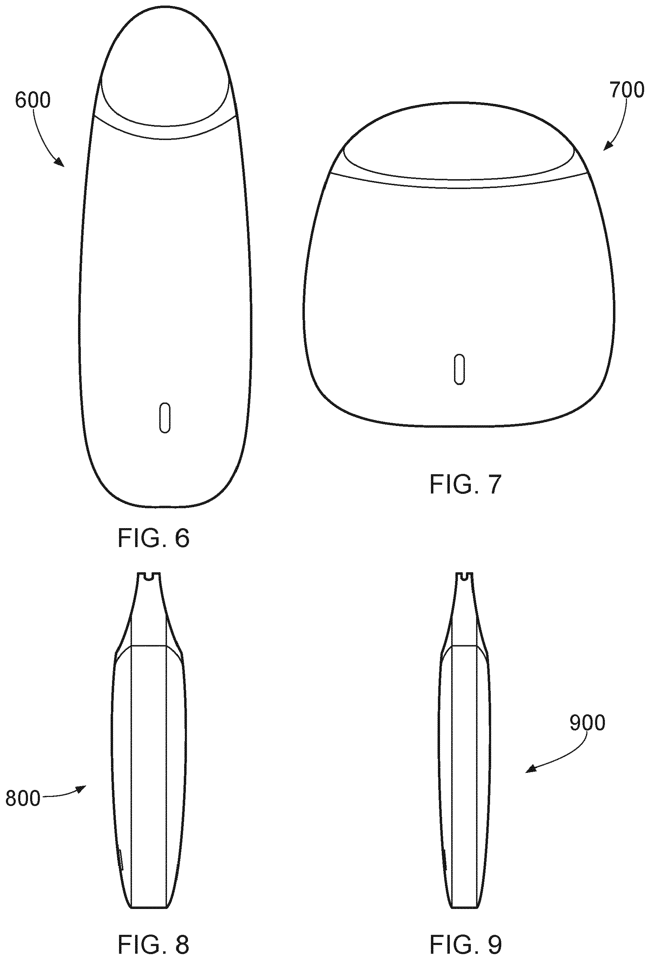

For example, in different configurations the characteristic outline of the device in the XY plane (i.e. perpendicular to the thickness direction) may be more elongate or less elongate than in the example represented in Figures 1 to 5. For example, Figure 6 schematically represents a view which is similar to, and will be understood from, the view of Figure 3A, but for a device 600 having a length which is around three times its width (as opposed to around double its width as in the example of Figures 1 to 5). Conversely, Figure 7 schematically represents a view which is similar to, and will be understood from, the view of Figure 3A, but for a device 700 having a length which is around the same as its width.

Similarly, in different configurations the characteristic relative thickness of the device along the Z direction may be greater or less than for the example represented in Figures 1 to 5. For example, Figure 8 schematically represents a view which is similar to, and will be understood from, the view of Figure 4A, but for a device 800 having a thickness which is around one-quarter of its length (as opposed to around one-fifth of its length as in the example of Figures 1 to 5). Conversely, Figure 9 schematically represents a view which is similar to, and will be understood from, the view of Figure 4A, but for a device 900 having a thickness which is around one-seventh of its length (as opposed to around one-fifth of its length as in the example of Figures 1 to 5).

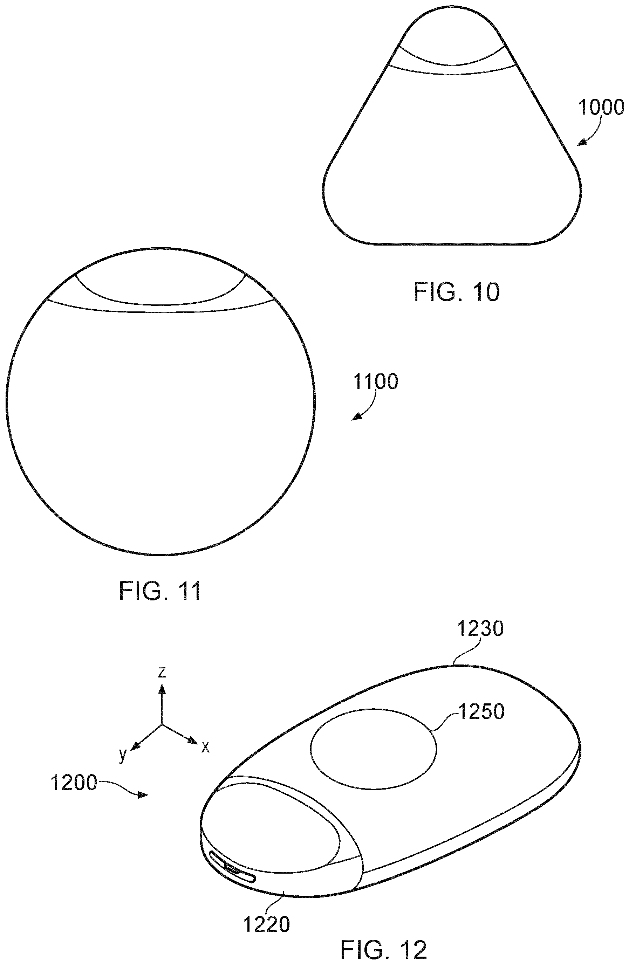

Furthermore, in other implementations devices in accordance with the principles described herein may have generally different outline shapes in a plane perpendicular to their width, and indeed in other planes. For example, Figure 10 schematically represents a view which is similar to, and will be understood from, the view of Figure 3A, but for a device 1000 having an overall shape in the plane perpendicular to its thickness which has the form of a generally rounded triangle. As another example, Figure 11 schematically represents a view which is similar to, and will be understood from, the view of Figure 3A, but for a device 1100 having an overall shape in a plane perpendicular to its thickness which has a generally circular form.

Figure 12 schematically represents a view which is similar to, and will be understood from, the view of the electronic cigarette device 100 represented in Figure 2, but showing a device 1200 having a slightly different overall shape, in particular, a shape which is even more rounded than that represented in Figure 2, for example in terms of the minimum radius of curvature for the outline of the device viewed in a plane perpendicular to the length (Y-) axis direction. As for the examples represented in Figures 1 to 11, the device 1200 represented in Figure 12 comprises a control unit part 1230 and a separable / replaceable cartridge part 1220. However, the device 1200 of Figure 12 also differs from the device 100 of Figure 2 in having a rounded, e.g. circular or elliptical, depression 1250 in the outer surface of the device, and in particular, in this example, in a surface shown uppermost in Figure 12 in a face of the control unit part 1230 which is generally perpendicular to the thickness direction (i.e. generally in the XY plane), the dimensions of the depression may be such that it has a width corresponding to around half the overall width W of the device 1200. However, in other implementations the depression may have a different size, for example the depression may extend in the width direction by an amount corresponding to between 0.2W and 0.8W, between 0.25W and 0.75W, between 0.3W and 0.7W, between 0.35W and 0.65W, between 0.4W and 0.6W, and between 0.45W and 0.65W. The depression / recess / indentation may have a broadly comparative extent in the width direction as in the length direction, or may have a different extent in the width direction as compared to the length direction. For example, the extent of the depression in the length direction may be greater than the extent of the depression in the width direction in some examples by a factor of around 1.5, or more. The depth of the depression along the thickness direction for the device may be around 3 mm. However, deeper or shallower depressions may also be used in accordance with other embodiments. For example, the depression may have a depth of between 1 mm and 5 mm, or more preferably between 2 mm and 4 mm. the inventors have recognised this kind of depression in the surface of the device can further facilitate a user's comfort in holding the device, for example by providing a more reliable grip. It will be appreciated more than one depression may be provided, for example depression may be provided symmetrically or otherwise on opposing surfaces of a device.lt will be appreciated that while the specific shape and size of a device in accordance with different implementations may vary, the same underlying principles which provide for a device which is convenient and comfortable to hold, whilst providing a relatively large volume for a given characteristic maximum extent, can be applied in the manner discussed herein.

Thus, in accordance with some embodiments of the present disclosure, a vapour provision device may be provided having a length greater than its thickness by a factor of at least 2, at least 2.5, at least 3, at least 3.5, at least 4, at least 4.5 or at least 5, and also having a width greater than its thickness by a factor of at least 2, at least 2.5, at least 3, at least 3.5, at least 4, at least 4.5 or at least 5. The length may be comparable to the width, or may be greater than the width, for example by a factor of at least 1.25, at least 1.3, at least 1.5, at least 2, at least 2.5, or at least 3.

In terms of some specific example dimensions, an electronic cigarette in accordance with some embodiments of the present disclosure may have a thickness which is less than 25 mm, less than 22mm, less than 20 mm, less than 18 mm, less than 16 mm, less than 14 mm, less than 12 mm, or less than 10 mm in conjunction / combination with a width which is greater than 20 mm, greater than 25 mm, greater than 30 mm, greater than 35 mm, greater than 40 mm, greater than 45 mm or greater than 50 mm, the particular combination of thickness and width in any given implementations being subject in some examples to the width being at least twice the thickness.

Thus, whereas the electronic cigarette 100 represented in Figures 1 to 5 is, for the sake of a concrete example, assumed to have an extent L x W x T of 70 mm x 35 mm x 14 mm, in another example, an electronic cigarette having a broadly similar overall shape may have an extent L x W x T of 90 mm x 40 mm x 16 mm. more generally, in accordance with some examples and electronic cigarette in accordance with the principles described herein may have a characteristic length between 60 mm and 100 mm, or more preferably between 70 mm and 90 mm and / or a characteristic width of between 30 mm and 45 mm, or more preferably between 35 mm and 40 mm and / or a characteristic thickness of between 12 mm and 20 mm, or more preferably between 15 mm and 17 mm.

In some cases it may be helpful to provide electronic cigarettes in accordance with the principles described herein with a length L of less than 120 mm, for example less than 110 mm, for example less than 100 mm, for example less than 90 mm, or less than 80 mm. This can be helpful, for example, to provide a relatively compact device, while adopting on the principles described herein to allow a relatively large battery to be used in correlation with the relatively compact device.

Furthermore, in accordance with some embodiments of the present disclosure, a device having a width W may have an outline shape in a plane perpendicular to its thickness which has a minimum radius of curvature of at least 0.1 W, at least 0.2 W, at least 0.3 W, at least 0.4 W or at least 0.5 W.

In terms of some specific example dimensions, a device in accordance with some embodiments of the present disclosure may have an outline shape in a plane perpendicular to its thickness which has a minimum radius of curvature of at least 3 mm, at least 4 mm, at least 5 mm, at least 6 mm, at least 7 mm, at least 8 mm, at least 9 mm or at least 10 mm.

As a consequence of the generally rounded form of devices in accordance with the principles described herein, it will be appreciated the areal extent of a device in accordance with embodiments of the disclosure in a plane perpendicular to the devices thickness T may be somewhat less than the product of the device's length and width in this plane (because of the rounding of the corners). For example, in some implementations, the areal extent of a device in a plane perpendicular to its thickness may be less than the product of its width and length in this plane by a factor of less than 0.95, less than 0.9, less than 0.85, and less than 0.8.

As noted above, the inventors have recognised these types of configuration can help provide for aerosol provision systems which can be more convenient and comfortable to use than existing devices. The overall characteristic scale of a device may furthermore be chosen to broadly match the overall characteristic scale of an average human palm to help facilitate a comfortable grip. What is more, configurations in accordance with the principles described herein can in some implementations provide devices which a user can hold more discreetly than existing devices, for example by allowing a user to comfortably close their hand around the device.

While some particular examples have been described above, it will be appreciated there are many modifications that could be made in accordance with other implementations.

For example, it will be appreciated vapour provision devices incorporating features such as those described above to help provide a device shape which is comfortable and convenient for user to hold may in some cases include further features to enhance user comfort during use.

For example, it can be seen from the side views of Figures 4A and 4B, as well as the end view of figure 5B, that the thickness of the mouthpiece 250 in the above described example reduces towards the end of the device which is intended to be received by a user's lips during use (i.e. the vapour outlet 19). Accordingly, the mouthpiece portion 250 in effect tapers down to a thickness which is less than the thickness T of the device around its centre, but which has a width which is only slightly less than the width of the overall device. For example, the thickness of the mouthpiece in the vicinity of the vapour outlet (i.e. at a position received between a user's lips during normal use) may be less than 0.8 T, less than 0.7 T, less than 0.6 T, less than 0.5 T or less than 0.4 T. The width of the mouthpiece in the vicinity of the vapour outlet (i.e. at a position received between a user's lips during normal use) may in some examples be greater than 0.3 W, greater than 0.4 W, greater than 0.5 W, greater than 0.6 W or greater than 0.7 W. In terms of absolute dimensions, in accordance with some examples the thickness of the mouthpiece in the vicinity of the vapour outlet (i.e. at a position received between a user's lips during normal use) may be less than 12 mm, less than 10 mm, less than 8 mm, or less than 6 mm. The width of the mouthpiece in the vicinity of the vapour outlet (i.e. at a position received between a user's lips during normal use) may in some examples be greater than 10 mm, greater than 15 mm, greater than 20 mm, greater than 25 mm or greater than 30 mm. This results in a shape which broadly matches the opening in a user's lips in both size and shape. This shape and sizing of the mouthpiece 250 can therefore help the lips of user to engage the mouthpiece for inhalation with less distortion from the normal resting position of the mouth - e.g. there is no need to purse the lips, as for a straw or conventional cigarette having a small circular mouthpiece. This can help make using the mouthpiece 250 of the e-cigarette 100 a more relaxing experience for some users, and also may help to ensure a more consistent seal between the mouth and the mouthpiece. The relatively gradual reduction in thickness of the mouthpiece towards the vapour outlet 19, as opposed to a steeper change, can also help with comfortably matching the profile of a user's lips in a relatively natural rest position.

Furthermore, it will be appreciated that whereas the above-described embodiments have primarily focused on an electrical heater based vaporiser for heating a source liquid, the same principles may be adopted in accordance with vaporisers based on other technologies, for example piezoelectric vibrator based vaporisers, and devices based on other aerosol precursor materials, for example solid materials, such as plant derived materials, such as tobacco derivative materials.

It will further be appreciated the various references to thickness, length, and width herein are intended to refer to characteristic indications of such parameters. For example, it will be appreciated that as a consequence of the generally rounded nature of devices in accordance with the principles described herein, devices may not have the same width at all positions along their length and across their thickness. Similarly, the other dimensions (length and thickness) may not be the same for all positions on the device, but may vary depending on where they are measured due to the generally rounded characteristics of the device. Accordingly, the terms length, width, thickness etc. Are intended to reflect characteristic measures of these dimensions, for example maximum or average values, or values at the centre of the device for these dimensions. Average values, may, for example be formed from the mean, mode or median values for a plurality of different sampling points across a device.

Thus, there has been described a vapour provision device comprising a vaporiser for generating a vapour from a vapour precursor material for inhalation by a user; wherein the device has a length L along a length direction, a thickness T along a thickness direction which is orthogonal to the length direction, and a width W along a width direction which is perpendicular to both the length direction and the thickness direction, wherein the width W and length L are both at least twice the thickness T, and wherein a minimum radius of curvature for a peripheral edge of the device in a plane perpendicular to the thickness direction is at least 0.1 W.

The various embodiments described herein are presented only to assist in understanding and teaching the claimed features. These embodiments are provided as a representative sample of embodiments only, and are not exhaustive and / or exclusive. It is to be understood that advantages, embodiments, examples, functions, features, structures, and / or other aspects described herein are not to be considered limitations on the scope of the invention as defined by the claims or limitations on equivalents to the claims, and that other embodiments may be utilised and modifications may be made without departing from the scope of the claimed invention. Various embodiments of the invention may suitably comprise, consist of, or consist essentially of, appropriate combinations of the disclosed elements, components, features, parts, steps, means, etc., other than those specifically described herein. In addition, this disclosure may include other inventions not presently claimed, but which may be claimed in future.

In order to address various issues and advance the art, this disclosure shows by way of illustration various embodiments in which the claimed invention(s) may be practiced. The advantages and features of the disclosure are of a representative sample of embodiments only, and are not exhaustive and / or exclusive. They are presented only to assist in understanding and to teach the claimed invention(s). It is to be understood that advantages, embodiments, examples, functions, features, structures, and / or other aspects of the disclosure are not to be considered limitations on the disclosure as defined by the claims or limitations on equivalents to the claims, and that other embodiments may be utilised and modifications may be made without departing from the scope of the claims. Various embodiments may suitably comprise, consist of, or consist essentially of, various combinations of the disclosed elements, components, features, parts, steps, means, etc. other than those specifically described herein, and it will thus be appreciated that features of the dependent claims may be combined with features of the independent claims in combinations other than those explicitly set out in the claims. The disclosure may include other inventions not presently claimed, but which may be claimed in future.

## Claims

1. A vapour provision device comprising a vaporiser for generating a vapour from a vapour precursor material for inhalation by a user; wherein the device has a length L along a length direction, a thickness T along a thickness direction which is orthogonal to the length direction, and a width W along a width direction which is perpendicular to both the length direction and the thickness direction, wherein the width W and length L are both at least twice the thickness T, and wherein a minimum radius of curvature R for a peripheral edge of the device in a plane perpendicular to the thickness direction is at least 0.1 times the width W.

2. The vapour provision device of claim 1, wherein the length L is greater than the thickness T by a factor of at least 2, at least 2.5, at least 3, at least 3.5, at least 4, at least 4.5, or at least 5; and / or wherein the width W is greater than the thickness T by a factor of at least 2, at least 2.5, at least 3, at least 3.5, at least 4, at least 4.5, or at least 5.

3. The vapour provision device of claim 1 or 2, wherein the length L is greater than the width by a factor of at least 1.25, at least 1.3, at least 1.5, at least 2, at least 2.5, or at least 3.

4. The vapour provision device of any of claims 1 to 3, wherein the thickness T is less than 25 mm, less than 22mm, less than 20 mm, less than 18 mm, less than 16 mm, less than 14 mm, less than 12 mm, or less than 10 mm; and / or wherein the width is greater than 20 mm, greater than 25 mm, greater than 30 mm, greater than 35 mm, greater than 40 mm, greater than 45 mm, or greater than 50 mm.

5. The vapour provision device of any of claims 1 to 4, wherein the length is less than 120 mm, less than 110 mm, less than 100 mm, less than 90 mm, or less than 80 mm.

6. The vapour provision device of any of claims 1 to 5, wherein L is between 60 mm and 100 mm, or more preferably L is between 70 mm and 90 mm; and / or W is between 30 mm and 45 mm, or more preferably between 35 mm and 40 mm; and / or T is between 12 mm and 20 mm, or more preferably between 15 mm and 17 mm.

7. The vapour provision device of any of claims 1 to 6, wherein the minimum radius of curvature R for a peripheral edge of the device in the plane perpendicular to the thickness direction is at least 0.2 times the width W, at least 0.3 times the width W, at least 0.4 times the width W, or at least 0.5 times the width W.

8. The vapour provision device of any of claims 1 to 7, wherein the minimum radius of curvature R for a peripheral edge of the device in the plane perpendicular to the thickness direction is at least 3 mm, at least 4 mm, at least 5 mm, at least 6 mm, at least 7 mm, at least 8 mm, at least 9 mm or at least 10 mm.

9. The vapour provision device of any of claims 1 to 8, wherein an areal extent of the device in the plane perpendicular to the thickness direction is less than the product of width and the length by a factor of less than 0.95, less than 0.9, less than 0.85, and less than 0.8.

10. The vapour provision device of any of claims 1 to 9, wherein at least one of the surfaces of the device perpendicular to the thickness direction is curved in the width direction along a majority of the width of the device; and / or wherein at least one of the surfaces of the device perpendicular to the thickness direction is curved in the length direction along a majority of the length of the device.

11. The vapour provision device of any of claims 1 to 10, wherein at least one of the sides of the device perpendicular to the width direction is curved in the length direction along a majority of the length of the device; and / or wherein at least one of the ends of the device perpendicular to the width direction is curved in the width direction along a majority of the width of the device.

12. The vapour provision device of any of claims 1 to 11, wherein the device comprises a control unit and a detachable cartridge, wherein the cartridge comprises the vapour precursor material and the control unit comprises a power supply for supplying power to the vaporiser to selectively generate the vapour from vapour precursor material, and optionally, wherein the detachable cartridge further comprises the vaporiser.

13. The vapour provision device of any of claims 1 to 12, wherein the vapour precursor material comprises a liquid formulation.

14. A vapour provision device comprising a vaporiser for generating a vapour from a vapour precursor material for inhalation by a user; wherein a majority of the outer surface of the device is curved.

15. A vapour provision device comprising a vaporiser for generating a vapour from a vapour precursor material, wherein the device has a length L along a length direction, a thickness T along a thickness direction which is orthogonal to the length direction, and a width W along a width direction which is perpendicular to both the length direction and the thickness direction, wherein the width W and length L are both at least twice the thickness T, and wherein a majority of the a peripheral edge of the device in a plane perpendicular to the thickness direction is curved.
